(19) Europäisches Patentamt
European Patent Office
Office européen des brevets

(11) Publication number: **0 340 759**
**A1**

(12) **EUROPEAN PATENT APPLICATION**

(21) Application number: 89108029.3

(22) Date of filing: 03.05.89

(51) Int. Cl.[4]: C07F 9/09, C07C 101/30

(30) Priority: 06.05.88 IT 2048288
10.05.88 IT 2051488
13.05.88 IT 2057988
13.05.88 IT 2058288
13.05.88 IT 2058388

(43) Date of publication of application:
08.11.89 Bulletin 89/45

(84) Designated Contracting States:
BE DE ES FR GB GR IT LU NL

(71) Applicant: MAGIS FARMACEUTICI S.p.A.
Via Cacciamali 34/36/38
I-25125 Brescia(IT)

(72) Inventor: Puricelli, Laura
Via Taramelli, 7
I-25100 Brescia(IT)

(74) Representative: Gervasi, Gemma et al
NOTARBARTOLO & GERVASI Srl Viale
Bianca Maria 33
I-20122 Milan(IT)

(54) **Salts of O-mono (di)-acetyl-glycero-mono-(bis)-(di)-phosphates of L-acetyl-carnitine and of L-acetyl carnitine esters.**

(57) Salts of O-mono(di)-acetyl glycero-mono-(bis)-(di) phosphates of L-acetyl-carnitine and of L-acetyl carnitine ester are described.

These acetyl derivatives are useful agent in the treatment of slow cerebral metabolism, senile and pre-senile psychomotor involution, depressive states, senile and pre-senile dementia and reduced cerebral blood flow caused by primitive cerebral ictus or cardiac affections.

EP 0 340 759 A1

# SALTS OF O-MONO (DI)-ACETYL-GLYCERO-MONO-(BIS)-(DI)-PHOSPHATES OF L-ACETYL-CARNITINE AND OF L-ACETYL CARNITINE ESTERS.

This invention relates to the salts of L-acetyl carnitine or its esters with the O-mono (di) acetyl-glycero-mono-(bis)-(di)phosphates and pharmaceutical forms which contain them.

More particularly the invention relates to the compound of the following general formula:

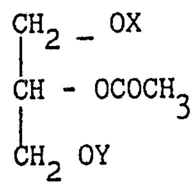

wherein Y = 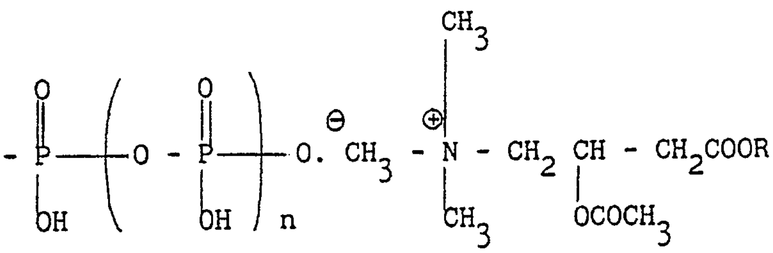

wherein n can be 0 or 1

R is a $C_1$ - $C_5$ alkylradical

X = Y, H, $COCH_3$

with the following proviso:

when X is H, n = 0

Generally, the compound of formula (I) is an extremely effective therapeutic agent in the treatment of slow cerebral metabolism, senile and pre-senile psychomotor involution, depressive states, senile and pre-senile dementia, and reduced cerebral blood flow caused by primitive cerebral ictus or cardiac affections.

The present invention therefore also relates to pharmaceutical compositions containing a therapeutically active quantity of the active principle dissolved in and/or mixed with a pharmaceutically acceptable solid or liquid support normally used the these compositions.

The active principle of the invention can be administered orally or can be injected intramuscularly.

For oral administration a non-toxic pharmaceutical preparation can be formed by mixing the active substance with the supports normally used for this purpose, such as starch, glucose, lactose, gelatin, magnesium stearate, glyceryl monostearate, talc, sodium chloride, propyleneglycol, ethanol etc.

The final products are therefore in capsule, tablet, pill, single-dose sachet or extemporaneous suspension or emulsion form.

In the case of an injectable formulation, the active principle is dissolved in suitable liquids.

In the treatment of slow cerebral metabolism, senile and presenile psychomotor involution, depressive states, senile and presenile dementia, or reduced cerebral blood flow caused by primitive cerebral ictus, cerebral ischemia or cardiac affections, the compound according to the invention can be administered in quantities ranging from 5 to 50 mg/kg per day in stepped doses, such as three or four times per day.

The pharmaceutical formulations of the invention can be administered in posological units containing for example 250, 500, 1000 or 1500 mg of active principle together with physiologically suitable supports and excipients.

The preparation process of these compounds is based on two basic reactions schemes respectively for the compounds of formula (I) wherein X = Y and for the compounds of formula (I) wherein X is H or $COCH_3$.

In the first case, starting from glycerol, through the reaction with two moles of $(C_6H_5)_3CCl$ in presence of pyridine we obtain the compound:

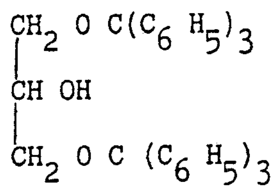

(II)

which is then reacted with acetic anhydride in presence of pyridine for obtaining the 2-O-acetyl derivative

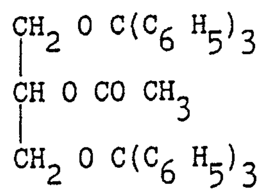

(III)

From this latter, through the reaction with HBr in acetic acid 2-acetyl-glycerol (IV)

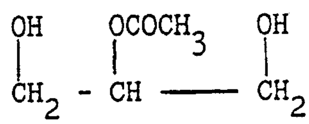

is obtained.

The following step is the reaction of (IV) with two moles of $ClPO(O\ C_6\ H_5)_2$ in presence of pyridine thus obtaining the compound:

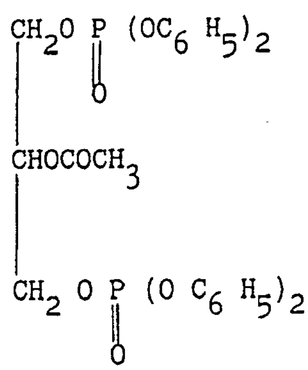

(V)

This compound is then reacted with $Ba\ (0H)_2$ in aqueous solution thus obtaining the Ba salt, the reaction mixture containing the excess of $Ba(O\ H)_2$ is treated with gaseous $CO_2$ until it becomes neutral to the phenolphtaleine and the $BaCO_3$ is filtered off. The aqueous solution of the Ba salt is treated with $H_2SO_4$ thus obtaining the 2-acetyl-glycerol-1,3-diphosphate:

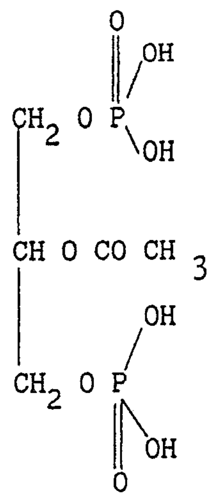

(VI)

In the case of the compounds of formula (I) wherein n = 0 the compound (VI) is directly salified in the ethyl alcohol with two moles of L-acetyl-carnitine or its esters whereas in the case n = 1 the compound (VI) is further reacted with $H_3PO_4$ in presence of pyridine and dicyclohexylcarbodimide thus obtaining the compound:

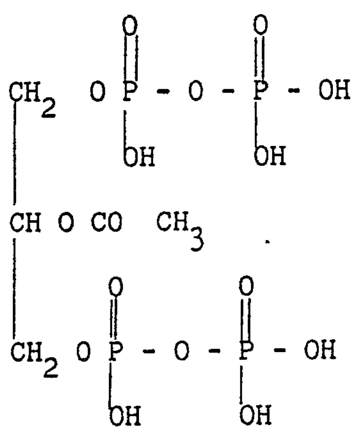

(VII)

which is then salified in ethyl alcohol with two moles of L-acetyl-carnitine or its esters.

In the second case, i.e. compounds of formula (I) wherein X is $-COCH_3$, starting from the 1,2-isopropyliden-glycerol:

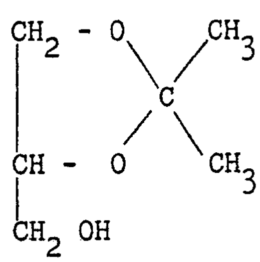

(VIII)

the reaction scheme is the following:

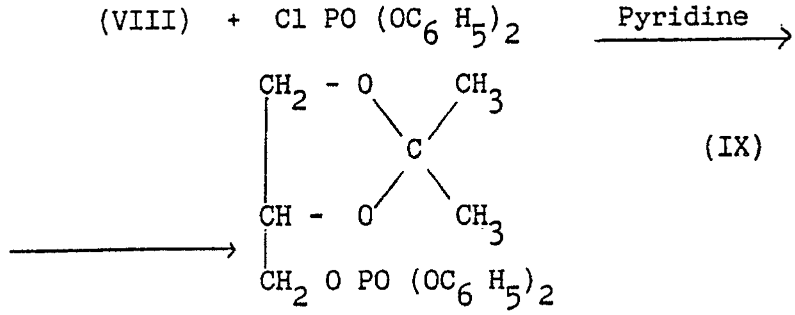


(IX)

The compound (IX) is reacted with Ba $(OH)_2$, $CO_2$ and $H_2SO_4$ as above reported for the first case, thus obtaining the compound:

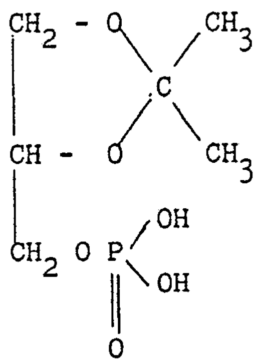

(X)

which hydrolized with HCl in aqueous solution, gives α-glycerophosphoric acid:

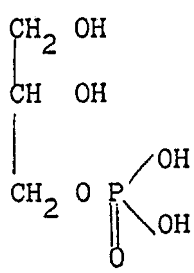

(XI)

The compound (XI) is completely acetylated in positions 1 and 2 by using acetic anhydride in presence of pyridine.

In the case of compounds of formula (I) wherein X = H, the compound (XI) is at first made to react with Cl C $(C_6H_5)_3$ in order to convert the -OH group in position 1 into the protected group -O C $(C_6H_5)_3$, then an acetylation with acetic anhydride in presence of pyridine occurs in position 2, thereafter the hydrolysis of the group $-OC(C_6H_5)_3$ is carried out by the use of H Br thus restoring the -O H group.

The monoacetyl- and respectively diacetyl- derivative is then salified in ethanol solution with one mol of L -acetyl-carnitine.

Finally, from the diacetyl derivative:

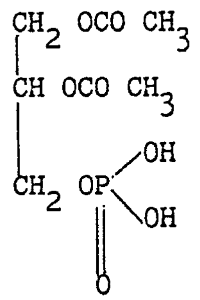

(XII)

it is possible to obtain the phosphoric acid derivative

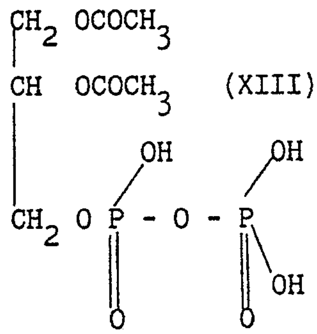

(XIII) through

the reaction of (XII) with $H_3PO_4$ + dicyclohexylcarbodimide in presence of pyridine.

Compound XIII is then salified with 1 mole of L-acetyl-carnitine.

Compounds of the general formula (I) wherein X = H or $-COCH_3$ can be obtained in form of the optical isomers D,L or DL form by using in their preparation α-glycerophosphoric acid (XI) in D or L forms, which can be obtained by the procedure described by Beer, Fischer J. Biol. Chem 128 pg. 491 (1939) pag.135, 321 (1940).

The compounds according to the present invention are the following:

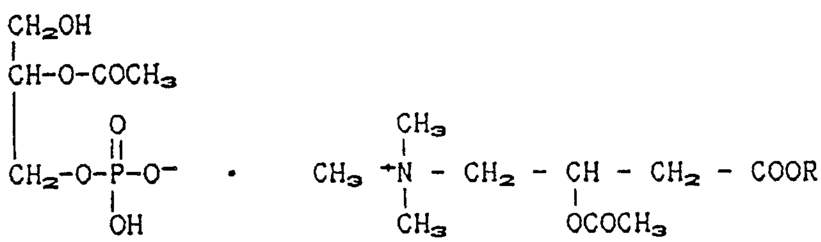

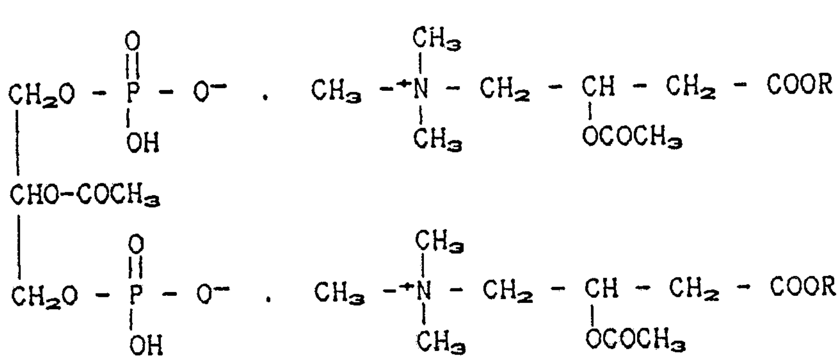

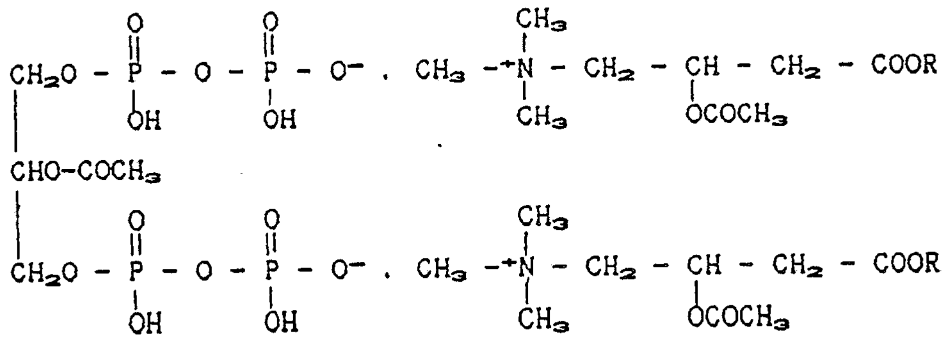

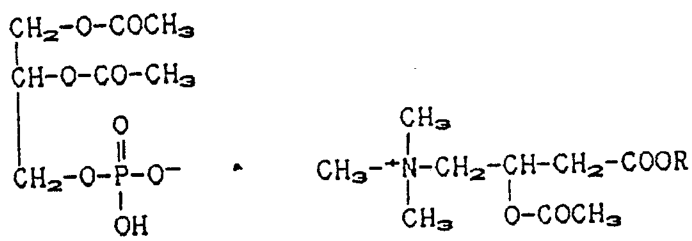

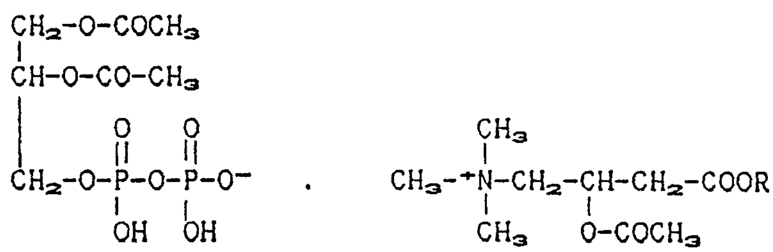

The pharmaco-biological characteristics of the compounds according to the present invention, namely their high activity and low toxicity, were evaluated with particular reference to acute toxicity, cardiokinetic activity, antiarrhythmic activity, its effects in terms of modifying the lipoprotein and hyper-excitability states and its capacity to return cholesterol and triglyceride levels to normal value.

The acute toxicity of the compounds of general formula (I) was studied on the mouse by C.S. Weill method [Biometr. J. 8, 249 (1952)].

The $LD_{50}$ exceeds 2000 mg/kg.

The cardiokinetic effect was studied on a Langerdoff isolated rabbit heart preparation.

The test shows that the compound has a positive inotropic effect calculated 10 minutes after interrupting the anoxic period.

The antiarrhythmic effect of the new compound was studied on the mouse by the procedure of P.W.N. Wangw and T. Holcow (P.W.N. Wangw, T.L. Holcow, Arch. Ind. Pharmacologin. 229, 219 1977).

The results show that the new compounds have beneficial effects on arrhythmia induced in the mouse by aconitine (5 mg/ml).

The effect of the new compounds in terms of modifying the lipoprotein state and the cholesterol and triglyceride levels, altered by oral administration of olive oil, was studied in normally fed rats treated orally with 15 mg/kg of olive oil one hour before oral administration of the compound at 300 mg/kg doses.

The new compound proved able to return the triglyceride, cholesterol and the α, β and pre-β lipoprotein fraction levels to normal values.

The activity of the new compound on the hyperexcitability state and on clonic contractions followed by depression induced by injection into the cerebellomedullary cistern of rats was studied by the method described by Irwing B. Frity in "Carnitine and its role in fatty acid metabolism", in "Advances in lipid research" I, 285-234, (1963) Academic Press.

Intracerebroventricular administration of the new compound causes pupil dilatation and markedly increased activity.

The following examples illustrate the invention but without limiting its scope.

EXAMPLE 1 2-acetyl-glycerol

9.21 g of glycerol and 57 g of triphenylmethylchloride are mixed with 100 ml of anhydrous pyridine in a flask fitted with a stirrer, and heated until completely dissolved. The solution is cooled to ambient temperature and 11 ml of acetic anhydride are added.

The mixture is allowed to stand for 12 hours and the solution then poured into 300 g of crushed ice, stirred until the ice has completely melted, acidified with hydrochloric acid and extracted with three 100 ml portions of chloroform.

The chloroform extracts are pooled, washed with an aqueous acid solution, dried with anhydrous sodium sulphate and then evaporated to dryness under vacuum.

The residue is dissolved in 200 ml of acetic acid.

The solution is cooled to about 10° C, 36 ml of a solution of dry hydrobromic acid in acetic acid added and the mixture agitated for about 45 seconds.

The triphenylmethylbromide which forms during the reaction is separated by filtration, and the filtrate transferred into 500 ml of a 20% sodium chloride solution. The aqueous phase is extracted repeatedly with ethyl acetate.

The organic phase is separated, dried with anhydrous magnesium sulphate and evaporated to dryness under vacuum. About 12 g of product are obtained.
Spectral analyses confirm the structure.

| Elementary analysis: | | |
|---|---|---|
| | C | H |
| calculated % | 44.769 | 7.513 |
| found % | 44.8 | 7.52 |

M.W. 134.15

EXAMPLE 2 2-acetyl-glycero-1.3-bisphosphate

300 ml of anhydrous pyridine, 57.6 g of diphenylphosphoryl chloride and 13.42 g of 2-acetyl-glycerol are transferred into a 1000 ml flask fitted with a stirrer and protected from moisture.

The mixture is agitated for two days at ambient temperature. After this time, 200 ml of water are added under agitation, the mixture transferred into a rotary flask and evaporated to dryness under vacuum (bath temperature 45-50° C).

The residue obtained is dissolved in 250 ml of alcohol, 400 ml of water and 280 g of barium hydroxide (octahydrate) are added to the solution, and the mixture heated under agitation for 60 minutes (from commencement of boiling).

When this time has elapsed, an energetic stream of carbon dioxide is fed into the solution until it becomes neutral to phenolphthaleine.

The mixture is filtered through a filter prepared with a light bed of dicalite, and the filter washed with 3 x 50 ml portions of water.

10% sulphuric acid is slowly added to the aqueous solution until pH 6.5 is attained. It is left standing for two hours and then filtered.

The filtrate is evaporated to dryness under vacuum in a rotary flask (bath temperature 45-50 ° C).

The residue is dissolved in 300 ml of anhydrous ethyl alcohol, filtered, and the filter washed with three 50 ml portions of anydrous ethyl alcohol.

300 ml of acetone are added slowly to the alkaline solution, the precipitate is filtered off and dried in an oven at 40 ° C under vacuum.

Spectral analyses confirm the structure.

| Elementary analysis: | | | |
|---|---|---|---|
| | C | H | P |
| calculated %<br>found % | 20.499<br>20.5 | 4.114<br>4.15 | 21.061<br>21.0 |

M.W. 294.09

EXAMPLE 3 L-acetyl carnitine 2-O-acetyl-glycero-1,3-bisphosphate

29.41 g of 2-O-acetyl-glycero-1,3-diphosphate are dissolved in 200 ml of absolute ethanol. When dissolved, 41 g of L-acetyl carnitine are added.

The solution is evaporated to dryness under vacuum. About 66.6 g of product are obtained.

Spectral analyses confirm the structure.

| Elementary analysis: | | | | |
|---|---|---|---|---|
| | C | H | N | P |
| - calculated %<br>- found % | 39.42<br>39.4 | 6.57<br>6.6 | 4<br>3.95 | 8.85<br>8.8 |

M.W. 700

EXAMPLE 4

Using the procedure of Example 3, the 2-O-acetyl glycero-1,3-phosphate of the L-acetyl carnitine methyl, ethyl, propyl, butyl and other esters are obtained.

EXAMPLE 5 2-acetyl glycero-1,3-bis diphosphate

2.94 g of 2-acetyl glycero-1,3-phosphate obtained according to example 2, 2.5 g of 85% phosphoric acid and 400 ml of pyridine are transferred into a 1 litre flask fitted with a stirrer. It is agitated until a solution forms and 100 g of D.C.C. are then added.

400 ml of water are added to the semisolid mixture, which is then agitated for 20 minutes and filtered.

The aqueous solution is extracted repeatedly with ether until pyridine is eliminated.

The filtered solution is reduced to 60 ml under vacuum at a temperature not exceeding 20 ° C.

The concentrated solution containing the diphosphoric derivative is dried by lyophilisation.

The residue is dissolved in alcohol and precipitated with ethyl ether operating at 0 ° C.

The process of precipitating from alcohol with ether is repeated three times.

About 2.5 g of product are obtained.

Spectral analyses confirm the structure.

| Elementary analysis: | | | |
|---|---|---|---|
| | C | H | P |
| - calculated %<br>- found % | 13.21<br>13.2 | 3.1<br>3.15 | 27.31<br>27.3 |

M.W. 454

EXAMPLE 6 L-acetyl-carnitine 2-acetyl-glycero-1,3-bis diphosphate

41.77 g of 2-acetyl-glycero-1,3-bis (diphosphate) are transferred into a 500 ml flask containing 200 ml of absolute alcohol cooled to 0° C in which 37.448 g of L-acetyl carnitine have been dissolved, and 300 ml of precooled acetone are added slowly to the solution.

The precipitate which forms is filtered off and dried in an oven under vacuum.

About 79 g of product are obtained.

Spectral analyses confirm the structure.

| Elementary analysis | | | | |
|---|---|---|---|---|
| | C | H | N | P |
| calculated %<br>found : | 32.09<br>32.10 | 5.6<br>5.55 | 3.25<br>3.28 | 14.42<br>14.45 |

M.W. 860

EXAMPLE 7

Using the procedure of Example 6, the 2-acetyl-glycero-1,3-bis diphosphate of the L-acetyl carnitine methyl, ethyl, propyl and butyl esters are obtained.

EXAMPLE 8 3-diphenylphosphoryl-1,2-isopropylidene glycerol

150 ml of anhydrous pyridine, 28.8 g of pure diphenylphosphoryl chloride and 13.216 g of salketal are fed into a 500 ml moisture-tight flask fitted with a stirrer.

The mixture is stirred for two days at ambient temperature. When this time has elapsed, 150 ml of water are added while stirring, the mixture is transferred into a rotary flask an evaporated to dryness under vacuum (bath temperature 45-50° C).

EXAMPLE 9 3-phosphoryl-1,2-isopropylidene-glycerol

The residue obtained from Example 8, containing 3-diphenylphosphoryl-1,2-isopropylidene-glycerol (about 35 g) and pyridine chloride, is dissolved in 100 ml of water. 350 ml of water and 140 g of barium hydroxide (octahydrate) are added to the solution. The mixture is heated under stirring for 65 minutes (from the commencement of boiling).

After this time, a strong carbon dioxide stream is fed into the solution until it becomes neutral to phenolphthaleine.

The mixture is filtered through a filter prepared with a light bed of dicalite. The filter is washed with three 50 ml portions of water.

The aqueous solution is extracted with ethyl ether to eliminate the phenol.

The aqueous solution is reduced to 150 ml under vacuum in a rotary flask. An equal volume of anhydrous ethyl alcohol is added and the mixture left stirring for two hours. The solution is filtered and the filter washed with small portions of 50% alcohol solution.

The mother liquors are pooled and 600 ml of anhydrous ethyl alcohol are slowly added.

The barium salt which precipitates is filtered off and washed with ethyl alcohol and then with ether. About 20-21 g of product are obtained.

The barium salt is dissolved in 150 ml of water and the solution pH corrected potentiometrically to 6.0-6.5 with a 10% sulphuric acid solution.

The mixture is left to stand for two hours and then filtered. The filtrate is evaporated to dryness under vacuum (bath temperature 45-50 °C). About 13 g of product are obtained.

Spectral analyses confirm the structure.

| Elementary analysis: | | | |
|---|---|---|---|
| | C | H | P |
| - calculated % | 29.85 | 7.014 | 15.395 |
| - found % | 29.9 | 7.1 | 15.28 |

M.W. 201.16

EXAMPLE 10 α-glycerophosphoric acid

22.5 g of 3-phosphoryl-1,2-isopropylidene glycerol are dissolved in 150 ml of 0.1 N hydrochloric acid and the mixture left stirring for 6 hours at ambient temperature.

The hydrochloric acid is neutralised with 15 ml of a 1 N sodium hydroxide solution. The solution is evaporated to dryness in a rotary flask (bath temperature 45-50 °C).

The residue is dissolved in 200 ml of absolute alcohol and the solution filtered and evaporated to dryness. The residue is then washed with acetone to obtain about 17 g of α-glycero phosphate.

| Elementary analysis: | | | |
|---|---|---|---|
| | C | H | P |
| - calculated % | 20.94 | 5.27 | 18.00 |
| - found % | 21.01 | 5.28 | 17.95 |

M.W. 172.01

EXAMPLE 11 1,2-di-O-acetyl-glycero-3-phosphoric acid

11.52 g of a-glycero phosphoric acid are dissolved in 50 g of anhydrous pyridine in a flask fitted with a stirrer. The mixture is heated moderately until a complete solution forms, after which it is cooled to ambient temperature and 15 ml of acetic anhydride added.

The solution is left standing for 12 hours after which it is poured into 200 g of crushed ice and stirred until the ice has dissolved. The precipitate which forms is filtered off, washed with acetone and dried in an oven. About 14 g of product are obtained.

Spectral analyses confirm the structure.

| Elementary analysis: | | | |
|---|---|---|---|
| | C | H | P |
| - calculated % | 32.82 | 5.144 | 12.10 |
| - found % | 32.85 | 5.2 | 12.05 |

M.W. 256.14

EXAMPLE 12 L-acetyl-carnitine 1,2-di-O-acetyl-glycerophosphate

25.614 g of 1,2-di-O-acetyl-glycero-3-phosphoric acid are dissolved in 100 ml of absolute ethanol and 10.724 g of L-acetyl carnitine are added to the solution. The solution is evaporated to dryness under vacuum. About 44.20 g of product are obtained.
Spectral analyses confirm the structure.

| Elementary analysis | | | | |
|---|---|---|---|---|
| | C | H | N | P |
| calculated % | 41.83 | 6.3 | 3.05 | 6.75 |
| found % | 41.85 | 6.25 | 3.1 | 6.8 |

M.W. 459.1

EXAMPLE 13

Using the procedure of Example 12, the 1,2-di-O-acetyl glycero3-phosphates of the L-acetyl carnitine methyl, ethyl, propyl and butyl esters are obtained.

EXAMPLE 14 1,2-di-O-acetyl-glycero-3-diphosphoric acid

2.561 g of 1,2-diacetyl-glycero-3-phosphoric acid, 1.153 g of 85 % phosphoric acid and 300 ml of pyridine are transferred into a 1 litre flask fitted with a stirrer. The mixture is agitated until a solution forms and 50 g of dicyclohexylcarbodiimide (D.C.C.) are then added. The mixture is agitated at 37° C for 5 days.

400 ml of water are added to the semisolid mixture, which is then agitated for 20 minutes and filtered. The aqueous solution is extracted repeatedly with ether until pyridine is eliminated.

The filtrate is reduced to 60 ml under vacuum at a temperature not exceeding 20° C.

The concentrated solution is passed through a column of Dowex 50 ion exchange resin (200-325 mesh in acid form) (length 24 cn x 8 cm diameter), and the column washed with water until the eluate is of neutral pH. The separated fraction containing the diphosphoric derivative is dried by lyophilisation (chromatograph system, ethanol: 1M ammonium acetate solution = 75:30 by vol.).

The residue is dissolved in alcohol and precipitated with ethyl ether operating at 0° C.

The process of precipitation from alcohol with ether is repeated three times. About 1.5 g of product are obtained.
Spectral analyses confirm the structure.

| Elementary analysis: | | | |
|---|---|---|---|
| | C | H | P |
| - calculated % | 25.092 | 4.198 | 18.428 |
| - found % | 25.1 | 4.2 | 18.1 |

M.W. 336.12

EXAMPLE 15 L-acetyl carnitine di-O-acetyl-glycero-3-diphosphate

33.612 g of diacetyl glycero-3-diphosphoric acid are transferred into a 500 ml flask containing 100 ml of ethyl alcohol cooled to 0° C in which 18.724 g of L-acetyl carnitine have been dissolved.

300 ml of acetone precooled to 0° C are aded to the solution, which is maintained at 0° C. The precipitate which forms is filtered off and dried in an oven under vacuum. About 52.30 g of product are obtained.
Spectral analyses confirm the structure.

| Elementary analysis | | | | |
|---|---|---|---|---|
| | C | H | N | P |
| calculated %<br>found % | 35.7<br>35.75 | 5.58<br>5.8 | 2.60<br>2.6 | 11.52<br>11.4 |

M.W. 538

EXAMPLE 16

Using the procedure of Example 15, the L-acetyl carnitine methyl, ethyl, propyl and butyl ester derivatives are obtained.

EXAMPLE 17 1-triphenylmethyl-2-acetyl-glycero 3-phosphate.

11.52 g of α-glycerophosphate obtained according to example 10 and 19.32 g of triphenylmethyl chloride are mixed with 50 ml of anhydrous pyridine in a flask fitted with a stirrer, and the mixture heated until completely dissolved.

It is cooled to ambient temperature and 7.5 ml of acetic anhydride added.

The solution is left to stand for 12 hours after which it is poured into 200 g of crushed ice and stirred until the ice has completely dissolved, the precipitate which forms being filtered off, dried and mashed in 200 ml of ether.

The insoluble part is filtered off and dissolved under hot conditions in 200 ml of absolute ethanol, the solution is decolorised with carbon, cooled, 200 ml of acetone added and the solution allowed to crystallize. The product is filtered off and dried in an oven.

About 12 g of product are obtained.

| Elementary analysis: | | | |
|---|---|---|---|
| | C | H | P |
| - calculated %<br>- found % | 63.15<br>63.21 | 5.5<br>5.45 | 6.8<br>6.9 |

M.W. 456

EXAMPLE 18 2-acetyl-glycero-3-phosphate

A solution of 46 g of 1-triphenylmethyl-2-acetyl-glycero-3-phosphate is dissolved under hot conditions in 200 ml of acetic acid. The solution is cooled to about 10 °C, 18 ml of a solution of dry hydrobromic acid in acetic acid are added and the mixture is stirred for about 45 seconds.

The triphenylmethyl bromide which forms during the reaction is separated by filtration and the filtrate transferred rapidly into 500 ml of cold water. The solution obtained is extracted repeatedly with ethyl ether. The aqueous phase which separates is evaporated to dryness under vacuum (bath temperature 40-45 °C).

The residue is taken up in 100 ml of 95% ethanol and heated until dissolved, the solution filtered and 100 ml of acetone are slowly added.

The precipitate which forms is filtered off and dried in an oven to obtain about 30 g of product.

| Spectral analysis: | | | |
|---|---|---|---|
| | C | H | P |
| - calculated %<br>- found % | 28<br>28.1 | 5.14<br>5 | 14.5<br>14.55 |

M.W. 214

EXAMPLE 19 L-acetyl carnitine 2-O-acetyl-glycero-phosphate

18.811 g of 2-acetyl-glycero-3-phosphate are dissolved in 100 ml of absolute ethanol. 18.73 g of L-acetyl carnitine are added and the solution evaporated to dryness under vacuum.

44.20 g of product are obtained.
Spectral analyses confirm the structure.

| Elementary analysis: | | | | |
|---|---|---|---|---|
| | C | H | N | P |
| - calculated %<br>- found % | 42.000<br>42.1 | 7<br>6.95 | 3.4987<br>3.5 | 7.75<br>7.61 |

M.W. 400.347

EXAMPLE 20

Using the procedure of Example 19, the 2-O-acetyl-glycerophosphate of the L-acetyl carnitine methyl, ethyl, propyl and butyl esters are obtained.

**Claims**

1) Salts of O-mono-(di)-acetyl-glycero-mono-(bis)- (di) phosphates of L-acetyl-carnitine and of L-acetyl carnitine esters having the following general formula (I)

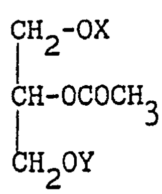

wherein Y = 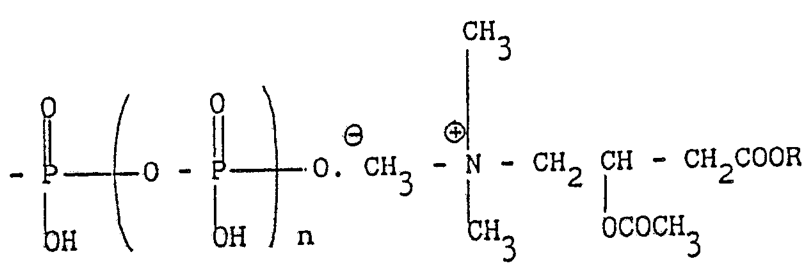 .

wherein n can be 0 or 1
R is a $C_1$-$C_5$ alkylradical
X = Y, H, $COCH_3$
with the following proviso:

when X is H, n = 0

2) A process for preparing the compounds according to claim 1, wherein X = Y which coprises the following steps:

a) glycerol is reacted with two moles of $(C_6H_5)_3$ C Cl in the presence of pyridine thus obtaining the compound:

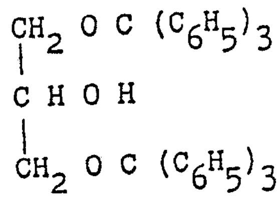

(II)

b) compound (II) is treated with acetic anhydride in presence of pyridine for obtaining the 2-acetyl-derivative (III);

c) from the 2-acetyl derivative (III) through reaction with HBr in acetic acid, 2-acetyl-glycerol (IV) is obtained;

d) 2-acetyl-glycerol (IV) by reacting with two moles of $Cl-PO(OC_6H_5)_2$ in presence of pyridine is converted into the compound 2-acetyl-glycero-1 ,3 bis (diphenyl phosphate), which is then reacted with $Ba(OH)_2$ in aqueous solution;

e) the reaction mixture containing the Ba salt and excess of $Ba(OH)_2$ is then treated with $CO_2$ and $Ba(CO_3)$ is filtered off, the Ba salt in solution is treated with $H_2SO_4$ thus obtaining the 2-acetyl-glycerol-1,3-diphosphate (VI);

f) directly salifying the compound (VI) with two moles of L-acetyl-carnitine or its esters for obtaining compounds wherein n = 0, or when n = 1;

g) the compound (VI) is further reacted with $H_3PO_4$ in presence of pyridine and dicyclohexyl carbodimide and the obtained compound:

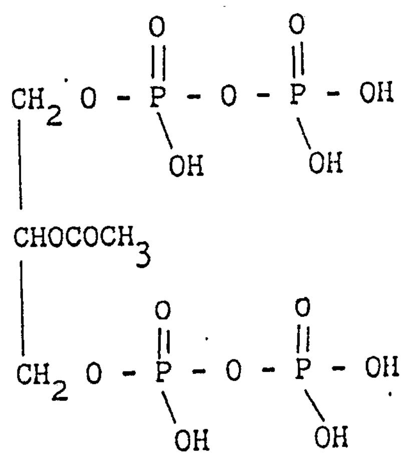

(VII)

is salified with two moles of L-carnitine or its esters.

3) A process for preparing the compounds according to claim 1, wherein X is - $COCH_3$, starting from α-glycerophosphoric acid, which comprises the following steps:

a) α-glycerophosphoric acid is completely acetylated with acetic anhydride in presence of pyridine and then, for obtaining compounds wherein X = $COCH_3$ and n = 0, is salified with L-carnitine or its esters, or when n = 1;

b) the completely acetylated α-glycerophosphoric acid is reacted with $H_3PO_4$ and dicyclohexylcarbodimide in the presence of pyridine, in order to obtain 1,2 diacetyl-3-diphosphoric acid which is then salified with L-acetyl-carnitine.

4) A process for preparing the compounds according to claim 1, wherein X = H, starting from α-glycerophosphoric acid, which comprises the following steps:

a) in the α-glycerophosphoric acid the OH in position 1 is protected through reaction with Cl $C(C_6H_5)_3$ and then acetylation is carried out in position 2;

b) restoring OH group in position 1 by treatment of reaction product of step a) with HBr;

c) salifying the 2-acetyl-glycerol-3-phosphate with L-acetyl-carnitine or its esters.

5) Pharmaceutical compositions for treating cardiac malfunctions, hyperlipoproteinemias, dyslipemias and slow cerebral metabolism, comprising a therapeutically effective quantity of the compound claimed in claim 1, in association with pharmacologically acceptable supports and adjuvants.

6) The pharmaceutical compositions according to claim 5, for oral or intramuscular administration.

7) The pharmaceutical compositions according to claim 5, containing fom 250 to 1.500 mg of active principle.

8) A therapeutic method for the treatment of slow cerebral metabolism, senile and pre-senile dementia, reduced cerebral blood flow caused by primitive cerebral ictus, cerebral ischemia or cardiac affections by administering in stepped doses the pharmaceutical compositions according to claim 5, so that the contained active principle ranges from 5 to 50 mg/kg per day.

9) A compound according to claim 1, which is 2-O-acetylglycero-1,3 bis-diphosphate-of L-acetyl carnitine or of L-acetyl carnitine esters of formula:

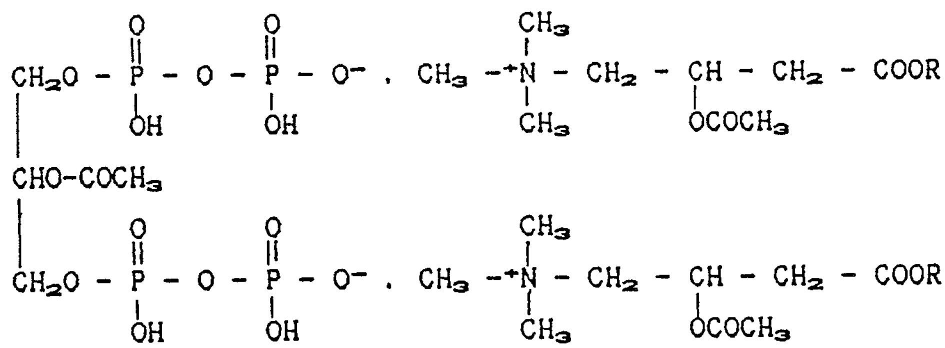

10) A compound according to claim 1, which is 1,2-di-O-acetyl glycero-3-phosphate of L-acetyl carnitine or of L-acetyl carnitine esters in DL, L or D form of formula:

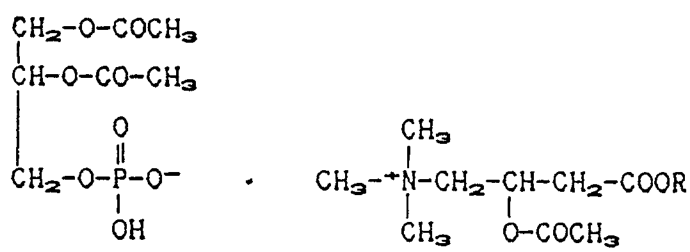

11) A compound according to claim 1, which is 1,2-di-O-acetyl glycero-3-di-phosphate of L-acetyl carnitine or of L-acetyl carnitine esters in DL, L or D form, of formula:

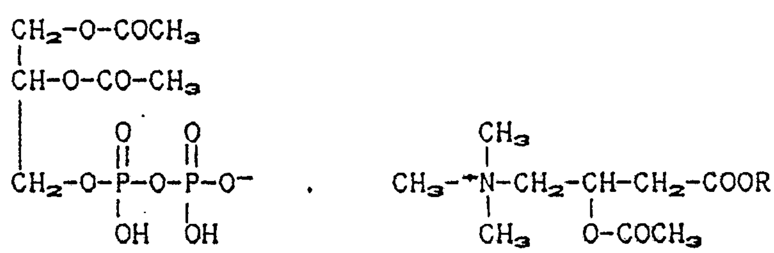

12) A compound according to claim 1, which is 2-O-acetylglycero-phosphate of L-acetyl carnitine or of its esters in DL, L or D form, of formula:

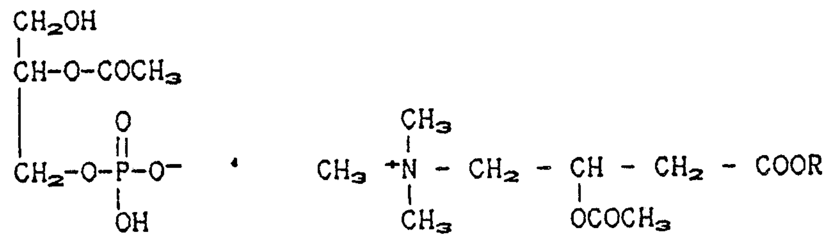

13) A compound according to claim 1, which is 2-O-acetylglycero-1,3-bis-phosphate of L-acetyl carnitine or of its esters, of general formula:

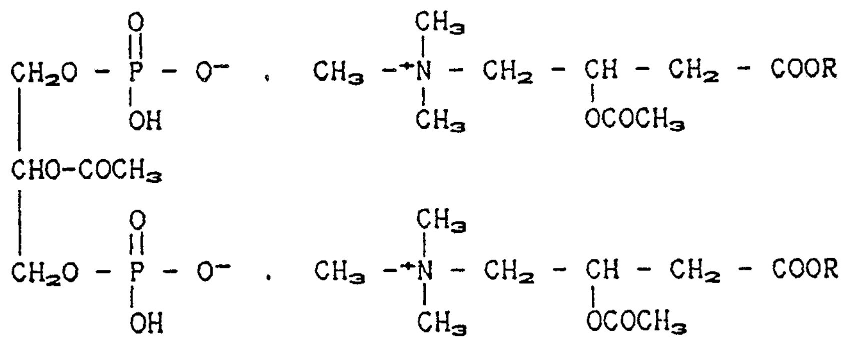

European Patent Office

# EUROPEAN SEARCH REPORT

Application Number

EP 89 10 8029

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-M- 6 345 (ETABLISSEMENTS FEVRIER, DECOISY, CHAMPION)<br>* Whole document *<br>--- | 1,5-8 | C 07 F 9/09<br>C 07 C 101/30 |
| A | EP-A-0 167 115 (MAGIS FARMACEUTICI S.p.A.)<br>* Whole document *<br>--- | 1,5-8 | |
| A | GB-A-2 045 612 (NIPPON SHOJI KAISHA LTD)<br>* Whole document *<br>----- | 1,5-8 | |

TECHNICAL FIELDS SEARCHED (Int. Cl.4)

C 07 F 9/00
C 07 C 101/00

The present search report has been drawn up for all claims

| Place of search | Date of completion of the search | Examiner |
|---|---|---|
| THE HAGUE | 10-08-1989 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document